# EUROPÄISCHE PATENTSCHRIFT

(11) **EP 3 589 784 B1**
(45) Veröffentlichungstag und Bekanntmachung des Hinweises auf die Patenterteilung: **28.04.2021**
(21) Anmeldenummer: 18707719.3
(22) Anmeldetag: 02.03.2018
(51) Int. Cl.: D04H 1/4258, A45D 44/00, A61Q 19/00, A61K 8/02, D04H 1/4391, D04H 3/013, D04H 3/018, D01F 2/06

(54) **VERWENDUNG EINER VISKOSEFASER**
USE OF A VISCOSE FIBRE
UTILISATION D'UNE FIBRE DE VISCOSE

(30) Priorität: 03.03.2017 EP 17159023
(43) Veröffentlichungstag der Anmeldung: 08.01.2020
(73) Patentinhaber: Kelheim Fibres GmbH, 93309 Kelheim (DE)
(72) Erfinder: BACHMANN, Alexander, 87490 Haldenwang (DE); BASEL, Sebastian, 86633 Neuburg/Donau (DE); BECK, Daniela, 89343 Jettingen-Scheppach (DE); BERNT, Ingo, 93053 Regensburg (DE)
(74) Vertreter: Nemec, Harald
(86) Internationale Anmeldenummer: PCT/EP2018/055151
(87) Internationale Veröffentlichungsnummer: WO 2018/158416

(56) Entgegenhaltungen:
- EP-A1- 2 301 380
- EP-A1- 2 599 900
- EP-A1- 2 860 307
- WO-A1-2015/154110

## Beschreibung

Die vorliegende Erfindung betrifft die Verwendung einer Viskosefaser zur Herstellung von kosmetischen Masken.

### Stand der Technik

Kosmetische Masken sind im Stand der Technik bekannt und bereits seit mindestens aus der Antike überliefert. Moderne kosmetische Tuchmasken haben allerdings nur wenig mit ihren antiken Gegenstücken gemeinsam und sind meist aus Vliesstoffen gefertigte High-tech Produkte, die in Serie produziert und in großen Stückzahlen vertrieben werden. Der weltweite Markt für kosmetische Tuchmasken ist stetig wachsend und gilt als einer der Wachstumsmotoren im Segment der kosmetischen Produkte.

Prinzipiell können eine Vielzahl verschiedenster Fasern für die Herstellung kosmetischer Tuchmasken verwendet werden, wobei die Eigenschaften der Ausgangsfasern entscheidend für das erzielte Ergebnis sind.

Beispielsweise weisen Standard-Viskosefasern eine krenulierte Faseroberfläche auf, an der Licht gestreut und reflektiert wird. Aus diesem Grund sind aus Standard-Viskosefasern hergestellte Papiere und Vliesstoffe intransparent.

Cellulosische Fasern, die nach dem Lyocellverfahren oder dem Cupro-Verfahren hergestellt wurden, weisen einen nahezu runden Querschnitt mit einer sehr glatten Oberfläche auf. Aus diesem Grund zeigen daraus hergestellte Papiere und Vliesstoffe eine höhere Transparenz. Aus diesen Fasern hergestellte Spunlace-Vliesstoffe mit einem Flächengewicht im Bereich von 30 - 65 g/m² weisen eine für die Anwendung in kosmetischen Gesichtsmasken ausreichende Transparenz auf.

Herkömmliche Viskosefasern werden aufgrund ihrer geringen Transparenz für die Anwendung in transparenten Gesichtsmasken nicht oder nur in geringem Ausmaß eingesetzt.

Lyocellfasern und Cupro-Fasern sind umgekehrt in ihrer Verfügbarkeit begrenzt und können den Marktbedarf nicht abdecken. Zudem ist das Wasserrückhaltevermögen (WRV) solcher Fasern niedriger, weshalb auch die Lotionsaufnahme und -abgabe gegenüber Viskose niedriger ist.

Kosmetische Tuchmasken sollen in der Anwendung transparent sein, damit der Anwender sieht, dass der Kontakt der Maske zur Haut komplett und blasenfrei ist, da nur dadurch eine gleichmässige Abgabe der Lotion auf die bedeckte Hautpartie gewährleistet ist. Dies ist insbesondere für in der Maske enthaltene Stoffe wichtig, die den Farbton der Haut verändern. Außerdem ergeben sich aus der Transparenz auch ästhetische und praktische Vorteile, da die Maske zum Beispiel im Büro oder öffentlichen Verkehrsmitteln getragen werden kann, da das Gesicht trotz Tragens einer Maske erkannt werden kann.

So ist aus der US 2008/069845 eine kosmetische Maske bekannt, deren der Haut zugewandte Schicht mindestens 10 % einer ultra-feinen Faser mit einem Titer von maximal 0,5 dtex enthält, vor allem um das Hautgefühl zu verbessern. Die genannte Offenbarung enthält jedoch keine Informationen über die Lichtdurchlässigkeit (Transparenz) der kosmetischen Maske. Selbiges gilt für die in der EP 1 813 167 offenbarten kosmetische Maske, die ebenfalls ultra-feine Fasern mit einem Titer von maximal 0,5 dtex enthält, um das Hautgefühl zu verbessern.

Ebenfalls bekannt ist aus dem Stand der Technik, wie in der US 2014/0352031 offenbart, eine kosmetische Maske, die aus einer Vliesstoffschicht und einer Nanofaserschicht mit einem hydrophilen Polymer besteht. Eine solche Maske soll vor allem das Hautgefühl verbessern und ein vorzeitiges Ablösen der einzelnen Maskenschichten (sog. Delamination) vermeiden. Genannte Offenbarung enthält allerdings keine Informationen über die Lichtdurchlässigkeit der Maske.

Aus der US 2014/0364365 ist eine kosmetische Maske bekannt, die aus Naturfasern besteht, welche mit einem hydrophilen Reagenz derivatisiert wurden um die Absorption von Hautpflegemittel zu verbessern. Diese Maske wird als transluzent und gelartig beschrieben.

Eine im nassen Zustand als transparent beschriebene Maske ist in der WO 2013/187404 bzw. der EP 2 860 307 A1 offenbart. Die Maske besteht aus einem Vliesstoff mit einem Flächengewicht zwischen 30 bis 100 g/m², der mindestens 30 % (w/w) einer transparenten Stapelfaser mit einer Faserlänge zwischen 20 und 70 mm umfasst. Die transparente Stapelfaser ist beispielsweise eine "solvent-spun"-Faser, also eine Lyocellfaser. Die Beimengung von Viskosefasern führt gemäß diesem Dokument zu einer Verringerung der Transparenz.

Eine nach Kontakt mit einem Benetzungsmittel ebenfalls als transparent beschriebene kosmetische Maske ist in der EP 2 384 734 offenbart. Als mögliche Fasertypen zur Herstellung des Vliesstoffs werden ganz generell sowohl natürliche, als auch synthetische Fasern genannt.

Zusätzlicher Stand der Technik zum Thema vorliegender Erfindung wird beispielsweise in der KR 10 614 126, der KR 2008/0051314, der KR 2009/0014693, der WO 2015/5046301, sowie der US 2016/0002859 zur Verfügung gestellt.

Es besteht nach wie vor die Aufgabe, Fasern aufzufinden, die sich gut zur Verwendung in der Herstellung hochtransparenter kosmetischer Masken eignen.

Bei Verwendung einer solchen Faser soll eine Maske resultieren, die sich blasenfrei an die Kontur der zu behandelten Hautpartie (z.B. Gesicht) anlegt. Weiters muss die Maske Lotion aufnehmen und an die Haut abgeben können.

Diese Aufgabe wird durch die erfindungsgemäße Verwendung einer Viskosefaser zur Herstellung einer transparenten kosmetischen Maske gelöst, wobei die Viskosefaser eine Flachfaser ist, wobei der Querschnitt der Viskosefaser ein Breiten- zu Dickenverhältnis von 6:1 bis 30:1 hat, und der Titer der Viskosefaser von 1,0 dtex bis 4 dtex liegt.

Bevorzugte Ausführungsformen der vorliegenden Erfindung werden in den Unteransprüchen angeführt.

### Kurze Beschreibung der Figuren

Figur 1 zeigt die Querschnitte von Fasern gemäß Beispiel 1 im Lichtmikroskop.
Figur 2 zeigt die Querschnitte von Fasern gemäß Beispiel 2 im Lichtmikroskop.
Figur 3 zeigt die Transparenz von Rapid-Koethen Papier, das aus 100% der Faser aus Beispiel 1.1 hergestellt wurde.
Figur 4 zeigt Querschnitte von Fasern gemäß Beispiel 3 im Lichtmikroskop.
Figur 5 zeigt die Transparenz von Rapid-Koethen Papier, das aus 80% der Faser gemäß Beispiel 4 mit 20% Zellstoff hergestellt wurde.

### Detaillierte Beschreibung der Erfindung

Es hat sich gezeigt, dass mit der Verwendung von Viskoseflachfasern, deren Querschnitt ein Breiten- zu Dickenverhältnis von 6:1 bis 30:1 hat, und deren Titer von 1,0 dtex bis 4 dtex beträgt, transparente kosmetische Masken mit hervorragenden Eigenschaften erhalten werden können.

Im Vergleich zu Standardviskosefasern wird eine verbesserte Transparenz der erhaltenen Masken erreicht. Im Vergleich zu Lyocellfasern und Cupro wird jedenfalls gleich gute oder auch verbesserte Transparenz der erhaltenen Masken erreicht. Zudem liegt die Maske gut an der zu behandelnden Hautfläche (z.B. Gesicht) an und weist im Vergleich zu Masken enthaltend Lyocell- oder Cuprofasern ein verbessertes Feuchtigkeitsmanagement (Wasserrückhaltevermögen, Lotionaufnahme und -abgabe) auf.

Eine transparente kosmetische Maske, die unter Verwendung der erfindungsgemäß spefizierten Viskoseflachfasern hergestellt wird, kann insbesondere einen Transparenzindex gemäß der unten dargestellten Messmethode von 1000 oder mehr, bevorzugt 1200 oder mehr aufweisen.

In einer besonderen Ausführungsform ist die erfindungsgemäß verwendete Viskosefaser dadurch gekennzeichnet, dass der Querschnitt ein Breiten- zu Dickenverhältnis von 10:1 bis 30:1, bevorzugt von 13:1 bis 20:1, noch mehr bevorzugt von 15:1 bis 19:1 und besonders bevorzugt von 17:1 bis 18:1 aufweist.

In einer weiteren bevorzugten Ausführungsform beträgt der Titer der verwendeten Faser von 2,0 dtex bis 4,2 dtex, bevorzugt von 2,2 und 3,8 dtex, besonders bevorzugt von 2,4 dtex bis 3,0 dtex, insbesondere von 2,4 bis 2,5 dtex.

In einer weiteren bevorzugten Ausführungsform ist die erfindungsgemäß verwendete Viskosefaser im Wesentlichen glatt und/oder transparent.

Unter "im Wesentlichen glatt" wird verstanden, dass die Faser abgesehen von ihren Randbereichen im wesentlichen keine Rillen in Längsrichtung aufweist, welche eine Rillentiefe von mehr als 10%, insbesondere mehr als 5% der Faserdicke haben. Als "Rillen" sind dabei die für Standardviskosefasern typischen, im Verhältnis zur Breite der Faser kleinen Einbuchtungen in Längsrichtung zu verstehen.

Viskoseflachfasern mit den obigen Eigenschaften sowie deren Herstellung werden in der Europäischen Patentanmeldung EP 2 599 900 A1 beschrieben und werden im Folgenden als "Faser 1" bezeichnet. Diese Fasern werden u.a. zur Herstellung von transparentem Papier vorgeschlagen. Allerdings sind bei Vliesen, wie sie zur Herstellung von kosmetischen Masken verwendet werden, die Abstände zwischen den einzelnen Fasern viel größer als bei Papier.

In einer weiteren bevorzugten Ausführungsform wird eine Viskosefaser verwendet, deren Querschnitt ein Breiten- zu Dickenverhältnis von 6:1 bis 15:1, bevorzugt von 8:1 bis 9:1 und besonders bevorzugt 9:1 aufweist.

Der Titer dieser Ausführungsform von erfindungsgemäß verwendeten Viskosefasern kann bevorzugt von 1,0 dtex bis 3,0 dtex, bevorzugt von 1,7 bis 2,4, besonders bevorzugt von 1,8 dtex bis weniger als 2 dtex, insbesondere 1,9 dtex betragen.

Eine solche Viskosefaser mit einem im Vergleich zur "Faser 1" etwas geringeren Breiten- zu Dickenverhältnis sowie einem etwas geringeren Titer wird im Folgenden als "Faser 2" bezeichnet.

Zur Herstellung von Fasern der Variante "Faser 2" mit einem Breiten- zu Dickenverhältnis von weniger als 10:1 kann das in der EP 2 599 900 A1 beschriebenen Verfahren unter Einsatz einer Schlitzdüse mit entsprechend modifizierten Maßen, z.B. 140 µm x 25 µm, verwendet werden.

In einer weiteren bevorzugten Ausführungsform wird die Viskosefaser zur Herstellung einer kosmetischen Maske in lotionsgetränkter Blatt(vlies)form benutzt.

Alternativ ist natürlich auch die Verwendung der Viskosefaser zur Herstellung einer kosmetischen Maske in Form eines trockenen Vliesstoffes möglich, der erst unmittelbar vor der Anwendung mit Lotion oder Wasser getränkt wird.

In einer weiteren bevorzugten Ausführungsform besteht die erfindungsgemäß verwendet Viskosefaser zu mehr als 98 % (w/w) aus Cellulose. Die Faser besteht somit im Wesentlichen aus Cellulose und enthält keine oder nur unwesentliche Anteile an anderen funktionellen Materialien, wie z.B. saugfähigen Polymeren.

In einer anderen Ausführungsform enthält die Viskosefaser ein saugfähiges Polymer in einem Anteil 2% bis 20%, bevorzugt 3% bis 10%, besonders bevorzugt 4% bis 7% (jeweils w/w). Das saugfähige Polymer kann dabei an der Oberfläche der Faser vorliegen und/oder in die Faser inkorporiert sein. Bei der zweiten Alternative wird das saugfähige Polymer der Spinnlösung der Faser vor dem Verspinnen zugemischt.

Bevorzugt ist in dieser Ausführungsform das saugfähige Polymer ausgewählt aus der Gruppe von Carboxymethylcellulose (CMC), modifizierter Stärke und Mischungen daraus.

Die erfindungsgemäß eingesetzte Viskosefaser weist bevorzugt eine Faserlänge von 2 mm bis 80 mm auf.

Bevorzugt erfolgt die erfindungsgemäße Verwendung von Viskosefasern zur Herstellung einer kosmetischen Maske in Gesichts-, Augen-, Finger-, Hand- oder Fußform.

Zur Herstellung der kosmetischen Maske kann eine Mischung der erfindungsgemäß verwendeten Viskoseflachfaser mit anderen Materialien, insbesondere anderen Fasermaterialien eingesetzt werden. In solchen Mischungen kann die erfindungsgemäß verwendete Viskoseflachfaser in einem Anteil von 50 Gew.% oder mehr, bevorzugt 80 Gew.% oder mehr, bezogen auf Gewicht der Maske eingesetzt werden.

Es sind verschiedene Herstellungsmethoden für Vliese aus den erfindungsgemäß verwendeten Viskosefasern möglich:
Bevorzugt werden Vliese bzw. Nonwovens aus den Fasern nach dem klassischen Spunlace-Verfahren hergestellt. Dafür eignen sich insbesondere Faserlängen von z.B. 34 bis 40 mm.

Ebenfalls möglich ist eine Herstellung durch ein Wetlaid-Verfahren mit anschließender Wasserstrahlverfestigung. Dafür sind Fasern mit kurzer Schnittlänge (bis 12 mm) gut geeignet.

Mit einem Krempel-Verfahren mit Luftstrom (Airlaid-Verfahren), wofür 20-25 mm lange Fasern benötigt werden, werden ebenfalls gute Ergebnisse erzielt.

In einer Variante kann man trockene Fasern mit Kurzschnittlänge (bis 12mm) auch im Airlaid-Verfahren mit anschließender Wasserstrahlverfestigung einsetzen.

Zusammengefasst können die erfindungsgemäß eingesetzten Fasern also eine Länge von 2 mm bis 80 mm besitzen und nach dem Spunlace-, Wetlaid-, Airlaid- und Krempel-Verfahren, jeweils mit oder ohne Wasserstrahlverfestigung verarbeitet werden.

Zur Herstellung der kosmetischen Maske wird zunächst das Vlies, z.B. in Form einer Vliesstoffrollenware (mit einem Flächengewichten von z.B. 15 - 100 g/m²), hergestellt.

Anschließend können optional mehrere weitere Lagen an Materialien übereinander gelegt werden, z. B. ein Polyester- oder Polypropylen(PP)-Schutzvlies, eine Stützfolie, etc. Danach wird die Form der zu bedeckenden Hautpartie (zB. eine Gesichtsform oder auch Augen-, Finger- oder Handformen) ausgestanzt. Danach wird die gestanzte Form gefaltet und einzeln verpackt.

Entweder vor dem Stanzen oder nach dem Stanzen wird eine Lotion aufgebracht. Möglich ist es auch, trockene Masken in Vertrieb zu bringen, auf die erst unmittelbar vor der Anwendung Lotion aufgebracht wird.

### Beispiele:

### Herstellung der Fasern und dazugehöriger Vlies-Muster

### Beispiel 1:

Bei diesem Beispiel handelt es sich um eine "Faser 1", die nach der Lehre der EP 2 599 900 A1 hergestellt wurde.

Die Viskose wurde durch eine Spinndüse mit schlitzförmigen Öffnungen mit einer Länge von 400 µm und einer Breite von 25 µm wie folgt versponnen und weiterbehandelt:
Abzug: 50 m/min, dies entsprach einem Düsenverzug von 2,7 Verstreckung (nach Verlassen des Spinnbades): 25 %
Viskose: Standardspinnviskose, enthaltend 4 Gew. % Polyethylenglycol (PEG) bezogen auf Cellulose.
Spinnbadzusammensetzung: 130 g/l H₂SO₄; restliche Bestandteile in dem in der EP 2 599 900 A1 angegebenen Bereich.
Nachbehandlung: Aufschwemmen, Waschen, Nachbehandlung, Schnitt auf 40 mm
Der Titer der Faser betrug 2,5 dtex.

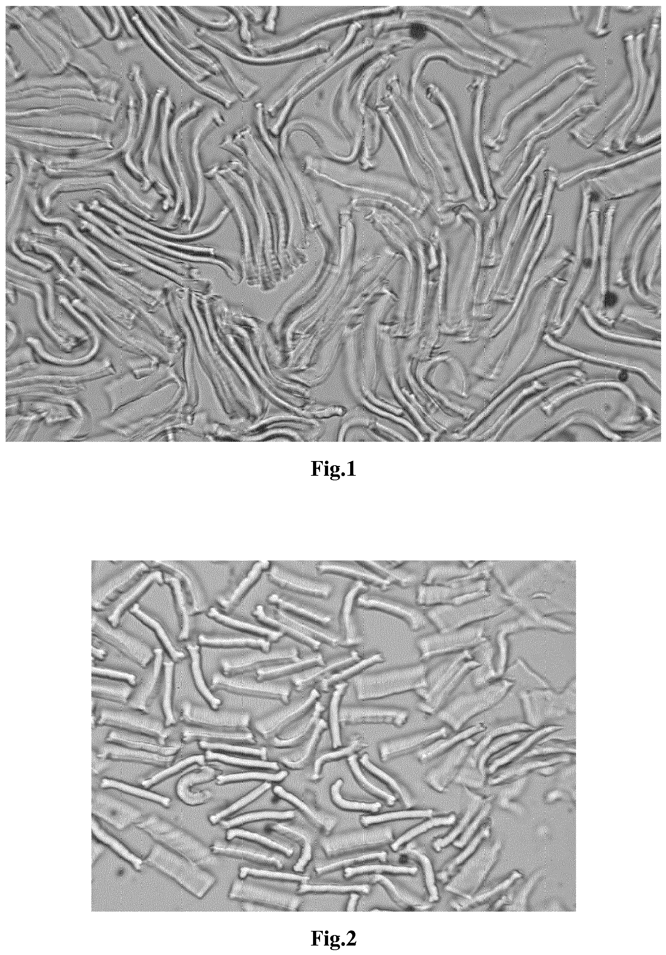

Figur 1 zeigt eine Mikroskopie-Aufnahme des Faserquerschnitts der erhaltenen Fasern. Die Faserquerschnitte sind sehr flach und dünn. Die beiden die Breitseite der Faser definierenden Flächen verlaufen praktisch über die gesamte Breite der Faser zueinander parallel. Lediglich an der Faserkante sind kleine Ausstülpungen vorhanden.

Die Breite B der Faser betrug etwa 55 µm, ihre Dicke D 3 µm. Daraus ergibt sich ein Verhältnis B:D von etwa 18:1.

Die Faser gemäß Beispiel 1 zeigt erhöhte Flachheit und Oberflächenglätte und gegenüber Standardviskosefasern erhöhte Transparenz.

Diese Fasern wurden zu Spunlace (Krempeln und Wasserstrahlverfestigung) mit einem Flächengewicht von 60 g/m² verarbeitet (im Folgenden als Muster 1 bezeichnet).

### Beispiel 1.1:

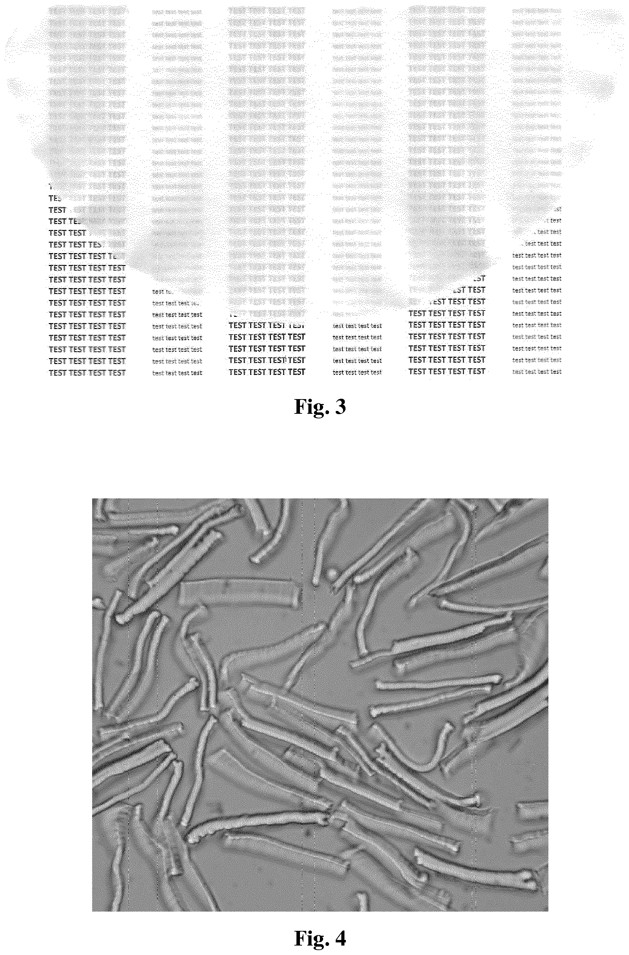

Viskosefasern wurden unter ähnlichen Bedingungen wie in Beispiel 1 versponnen, allerdings wurde die Faser zu 4 mm Kurzschnitt geschnitten. Ein Papiermuster 1.1 (Rapid-Köthen Blatt mit 80 g/m² aus 100 % der Faser), welches ohne Verwendung von Zusatzstoffen hergestellt wurde, zeigt bereits eine gute Transparenz (Figur 3, Vergleich der Intensität des Textes mit und ohne Abdeckung durch das Papiermuster).

### Beispiel 2:

Hier handelt es sich um eine Faser der Type "Faser 2". Diese wurde ebenfalls nach den Bedingungen der EP 2 599 900 hergestellt, soferne dies nicht im Folgenden anders angegeben ist:
Die Viskose wurde durch eine Spinndüse mit schlitzförmigen Öffnungen mit einer Länge (L) von 140 µm und einer Breite (B) von 25 µm (L:B = 5,6 :1) wie folgt versponnen und weiterbehandelt:
Abzug: 55 m/min, dies entsprach einem Düsenverzug von 2,2 Verstreckung (nach Verlassen des Spinnbades): 20 %
Viskose: Standardspinnviskose, enthaltend 3,8 Gew.% Polyethylenglycol (PEG) bezogen auf Cellulose.
Spinnbadzusammensetzung: 120 g/l H₂SO₄; restliche Bestandteile im Bereich gemäß EP 2 599 900.
Nachbehandlung: Aufschwemmen, Waschen, Nachbehandlung, Schnitt auf 40 mm
Der Titer der Faser betrug 1,9 dtex.

Querschnitte der so erhaltenen Fasern sind in der Figur 2 dargestellt und zeigen im Verhältnis zu Beispiel 1 ein geringeres Verhältnis von Breite B zu Dicke D. Mit einer Breite B der Faser von 32 µm und Dicke D von 4 µm ergibt sich ein Verhältnis B:D von 8:1. Die Oberfläche der Faser weist eine weitestgehend glatte Oberfläche auf.

Die Faser ist wesentlich glatter als im Stand der Technik bekannte reguläre Viloft®-Faser (eine herkömmlich hergestellte Viskoseflachfaser) mit gleichen Dimensionen, aber nicht so glatt wie die "Faser 1" .

Diese Fasern wurden zu mittels Spunlace-Verfahren (Krempeln und Wasserstrahlverfestigung) zu Vliesen mit einem Flächengewicht von etwa 60 g/m² verarbeitet (im Folgenden als Muster 2 bezeichnet).

### Beispiel 3 (Beispiel 1 mit CMC ):

Die Viskosefasern wurden unter ähnlichen Bedingungen wie in Beispiel 1 versponnen, allerdings enthielt die Standardspinnviskose neben 3,8 Gew. % Polyethylenglycol (PEG) bezogen auf Cellulose zusätzlich 17 Gew. % Carboxymethylcellulose.

Die Faserquerschnitte sind sehr flach und dünn (siehe Figur 4). Die beiden die Breitseite der Faser definierenden Flächen verlaufen praktisch über die gesamte Breite der Faser zueinander parallel. Lediglich an der Faserkante sind kleine Ausstülpungen vorhanden. Die Oberfläche ist weniger glatt als die Faser aus Beispiel 1. Die Transparenz ist jedoch im feuchten Zustand aufgrund der erhöhten Wasseraufnahme durch CMC vergleichbar. Die Breite B der Faser betrug 52 µm, ihre Dicke D 3 µm. Daraus ergibt sich ein Verhältnis B:D von 17:1.

Diese Fasern wurden zu Spunlace (Krempeln und Wasserstrahlverfestigung) mit einem Flächengewicht von 60 g/m² verarbeitet.

### Beispiel 4: (Beispiel 2 mit CMC):

Die Herstellung dieser Faser entspricht jener von Beispiel 2, mit dem Unterschied, dass bei dieser Faser noch zusätzlich 5 Gew.% Carboxymethylcellulose eingearbeitet wurden:

Die Viskose wurde durch eine Spinndüse mit schlitzförmigen Öffnungen mit einer Länge von 140 µm und einer Breite von 25 µm wie folgt versponnen und weiterbehandelt:
Abzug: 55 m/min, dies entsprach einem Düsenverzug von 2,2
Verstreckung (nach Verlassen des Spinnbades): 20 %
Viskose: Standardspinnviskose, enthaltend 3,8 Gew.% Polyethylenglycol (PEG) und zusätzlich 5 Gew. % Carboxymethylcellulose bezogen auf Cellulose.
Spinnbadzusammensetzung: 120 g/l H₂SO₄; restliche Bestandteile im Bereich gemäß EP 2 599 900.
Nachbehandlung: Aufschwemmen, Waschen, Nachbehandlung, Schnitt auf 40 mm
Der Titer der Faser betrug 1,9 dtex.

Die Querschnitte der so erhaltenen Fasern zeigen ein geringeres Verhältnis von Breite B zu Dicke D. Mit einer Breite B der Faser von 32 µm und Dicke D von 5 µm ergibt sich ein Verhältnis B:D von 6,3:1.

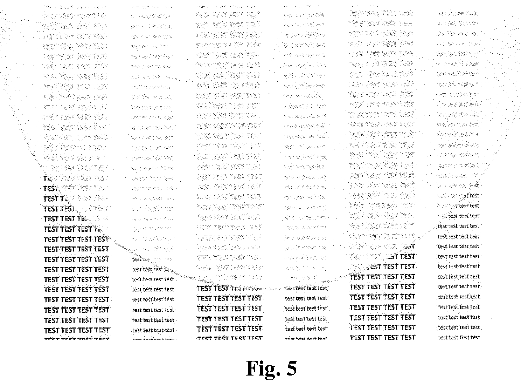

Für die Analytik wurden die Fasern zu 4 mm Kurzschnitt geschnitten und zu einem Rapid-Koethen-Papier zusammen mit 20 % Zellstoff verarbeitet. Die Transparenz dieses Papiers ist in Figur 5 dargestellt.

### Beispiel 5 (Beispiel 2 mit höherem Einsatz an PEG):

Es wurde bei ansonsten gleichen Versuchsparametern eine Faser gemäß den Bedingungen des Beispiels 2eine Faser 2 ("Faser 2") hergestellt, mit der Ausnahme dass der Gehalt an eingesetztem Polyethylenglycol (PEG) 9,5 Gew.% bezogen auf Cellulose betrug.

Die Transparenz der so erhaltenen Fasern sowie daraus hergestellter Vliese war höher als bei den Fasern gemäß Beispiel 2, aber geringer als bei den "Fasern 1".

### Ausprüfung der Muster in Vliesform und als Papier:

Hier ist vorauszuschicken, dass eine Bewertung der Fasern im Papier lediglich als Schnellmethode oder Vorhersage verwendet wird. Hier sind die Fasern im Wesentlichen coplanar zur Papierebene ausgerichtet. Im Vliessstoff sind die Fasern teils auch verdrillt oder stehen senkrecht oder diagonal zur Papierebene. Eine Messung am Vliesstoff ist daher zu bevorzugen. Eine Messung am Papier ist aber schnell möglich und erlaubt eine erste Einschätzung der Transparenz der Faser.

Die Messung erfolgte am Datacolor® SF600. Zunächst wurde das Gerät auf Schwarz- und Weißstandard kalibriert (Schwarzstandard: Datacolor; Weißstandard: Datacolor white Calibration Standard; Serial No 6060). Die Transparenz wurde indirekt über die Farbstärke (Helligkeit, Weißgrad) eines schwarzen Hintergrundes hinter einem der Muster gemessen. Es wurde jeweils eine 5-fach Bestimmung durchgeführt. Die Muster wurden nass vermessen. Dafür wurden die Vlies-Muster auf DIN A4 zugeschnitten und in eine offene Klarsichthülle (Leitz® Typ 4744 Overhead) gelegt. Anschließend wurden die Blätter bis zur Sättigung angefeuchtet und für 60 Sekunden abtropfen gelassen. Die Klarsichthülle wird geschlossen und die Messung der Farbstärke ("Farbstärken-Differenz" im Messprotokoll) wird mit einer Messblende mit 30 mm Durchmesser durchgeführt. Das Flächengewicht der Vliesstoffe wurde gemessen.

**Tabelle 1: Transparenzindex von verschiedenen Vlies-Mustern**

| | **Farbstärke** | **Flächengewicht (FG) in g/m²** | **Transparenzindex** (Farbstärke*FG/1000) | **Vergleich zu Standardviskose** |
|---|---|---|---|---|
| kommerzielle Gesichtsmaske aus LyocellFaser | 38000 | 30 | 1140,0 | 152% |
| Standardviskose Spunlace | 14388 | 52 | 748,2 | 100% |
| Muster 1 (Spunlace-Vliesstoff aus Faser 1) | 29506 | 55 | 1622,8 | 217% |
| Muster 2 (Spunlace-Vliesstoff aus Faser 2) | 19284 | 68 | 1311,3 | 175% |

| | | | | |
|---|---|---|---|---|
| * Transparenzindex = Farbstärke * Flächengewicht (FG) /1000 | | | | |

Der Transparenzindex zeigt, dass mittels Flachviskosefasern Vliesstoffe mit einer höheren Transparenz als aus dem Marktstandard Lyocell hergestellt werden können. Gegenüber Standardviskose kann die Transparenz auf mehr als das Doppelte gesteigert werden.

**Tabelle 2: Transparenzindex von verschiedenen Papier-Mustern.**

| 80 % Faser, 20 % Zellstoff | Farb stärke | Flächengewicht (FG) g/m² | Transparenzindex (Farbstärke*FG/1000) | Vergleich zu Standardviskose |
|---|---|---|---|---|
| Standardviskose 1,7 dtex | 5003 | 80,0 | 400,2 | 100% |
| Beispiele 1/1.1 | 11070 | 80,0 | 885,6 | 221% |
| Beispiel 2 | 9003 | 80,0 | 720,2 | 180% |
| Beispiel 4 | 11243 | 78,7 | 884,4 | 221% |

Der Transparenzindex zeigt, dass mittels Flachviskosefasern Flächen mit einer höheren Transparenz als mit Standardviskose hergestellt werden können.

Es zeigt sich dabei qualitativ eine recht gute Übereinstimmung zwischen den Ergebnissen bei Vliesstoffen (Tabelle 1) und bei Papier (Tabelle 2).

## Patentansprüche

1. Verwendung einer Viskosefaser zur Herstellung einer transparenten kosmetischen Maske, **dadurch gekennzeichnet, dass**
- die Viskosefaser eine Flachfaser ist
- der Querschnitt der Viskosefaser ein Breiten- zu Dickenverhältnis von 6:1 bis 30:1 hat, und
- der Titer der Viskosefaser von 1,0 dtex bis 4 dtex beträgt.

2. Verwendung einer Viskosefaser nach Anspruch 1, **dadurch gekennzeichnet, dass** der Querschnitt ein Breiten- zu Dickenverhältnis von 10:1 bis 30:1, bevorzugt von 13:1 bis 20:1, noch mehr bevorzugt von 15:1 bis 19:1 aufweist und besonders bevorzugt von 17:1 bis 18:1 aufweist.

3. Verwendung einer Viskosefaser nach Anspruch 1 oder 2, **dadurch gekennzeichnet, dass** der Titer der Faser(n) von 2,0 dtex bis 4,2 dtex, bevorzugt von 2,2 bis 3,8 dtex, besonders bevorzugt von 2,4 dtex bis 3,0 dtex, insbesondere von 2,4 bis 2,5 dtex beträgt.

4. Verwendung einer Viskosefaser nach einem der Ansprüche 2 oder 3, **dadurch gekennzeichnet, dass** sie im Wesentlichen glatt ist.

5. Verwendung einer Viskosefaser nach einem der Ansprüche 2 bis 4, **dadurch gekennzeichnet, dass** sie im Wesentlichen transparent ist.

6. Verwendung einer Viskosefaser nach Anspruch 1, **dadurch gekennzeichnet, dass** der Querschnitt ein Breiten- zu Dickenverhältnis von 6:1 bis 15:1, bevorzugt von 8:1 bis 10:1 und besonders bevorzugt von 8:1 bis 9: 1 aufweist.

7. Verwendung einer Viskosefaser nach Anspruch 6, **dadurch gekennzeichnet, dass** der Titer der Faser(n) von 1,0 dtex bis 3,0 dtex, bevorzugt von 1,7 bis 2,4, besonders bevorzugt von 1,8 dtex bis weniger als 2,0 dtex, insbesondere von 1,8 bis 1,9 dtex beträgt.

8. Verwendung einer Viskosefaser nach einem der vorhergehenden Ansprüche, **dadurch gekennzeichnet, dass** sie zur Herstellung einer kosmetischen Maske in lotionsgetränkter Blatt(vlies)form benutzt wird.

9. Verwendung einer Viskosefaser nach einem der vorhergehenden Ansprüche, **dadurch gekennzeichnet, dass** sie zu mehr als 98 % (w/w) aus Cellulose besteht.

10. Verwendung einer Viskosefaser nach einem der Ansprüche 1 bis 9, **dadurch gekennzeichnet, dass** sie ein saugfähiges Polymer in einem Anteil 2% bis 20% (w/w), bevorzugt 3% bis 10% (w/w), besonders bevorzugt 4% bis 7 % (w/w) enthält.

11. Verwendung gemäß Anspruch 10, **dadurch gekennzeichnet, dass** das saugfähige Polymer ein Polymer ausgewählt aus der Gruppe bestehend aus Carboxymethylcellulose (CMC), modifizierte Stärken und Mischungen davon ist.

12. Verwendung einer Viskosefaser nach einem der vorhergehenden Ansprüche, **dadurch gekennzeichnet, dass** sie eine Faserlänge von 2 mm bis 80 mm aufweist.

13. Verwendung einer Viskosefaser nach einem der vorhergehenden Ansprüche zur Herstellung einer kosmetischen Maske in Gesichts-, Augen-, Finger-, Hand- oder Fußform.

## Claims

1. The use of a viscose fibre for the production of a transparent cosmetic mask, **characterized in that**
- the viscose fibre is a flat fibre,
- the cross section of the viscose fibre has a width-to-thickness ratio of 6:1 to 30:1, and
- the titre of the viscose fibre ranges from 1.0 dtex to 4 dtex.

2. The use of a viscose fibre according to claim 1, **characterized in that** the cross section has a width-to-thickness ratio of 10:1 to 30:1, preferably of 13:1 to 20:1, more preferably of 15:1 to 19:1 and particularly preferably of 17:1 to 18:1.

3. The use of a viscose fibre according to claim 1 or 2, **characterized in that** the titre of the fibre(s) ranges from 2.0 dtex to 4.2 dtex, preferably from 2.2 to 3.8 dtex, particularly preferably from 2.4 dtex to 3.0 dtex, in particular from 2.4 to 2.5 dtex.

4. The use of a viscose fibre according to any of claims 2 or 3, **characterized in that** it is essentially smooth.

5. The use of a viscose fibre according to any of claims 2 to 4, **characterized in that** it is essentially transparent.

6. The use of a viscose fibre according to claim 1, **characterized in that** the cross section has a width-to-thickness ratio of 6:1 to 15:1, preferably of 8:1 to 10:1 and particularly preferably of 8:1 to 9:1.

7. The use of a viscose fibre according to claim 6, **characterized in that** the titre of the fibre(s) ranges from 1.0 dtex to 3.0 dtex, preferably from 1.7 to 2.4, particularly preferably from 1.8 dtex to less than 2.0 dtex, in particular from 1.8 to 1.9 dtex.

8. The use of a viscose fibre according to any of the preceding claims, **characterized in that** it is used for the production of a cosmetic mask in the form of a lotion-impregnated sheet (nonwoven).

9. The use of a viscose fibre according to any of the preceding claims, **characterized in that** it consists of cellulose by more than 98% (w/w).

10. The use of a viscose fibre according to any of claims 1 to 9, **characterized in that** it contains an absorbent polymer in a proportion of 2% to 20% (w/w), preferably of 3% to 10% (w/w), particularly preferably of 4% to 7% (w/w).

11. The use according to claim 10, **characterized in that** the absorbent polymer is a polymer selected from the group consisting of carboxymethyl cellulose (CMC), modified starches and mixtures thereof.

12. The use of a viscose fibre according to any of the preceding claims, **characterized in that** it has a fibre length of 2 mm to 80 mm.

13. The use of a viscose fibre according to any of the preceding claims for the production of a cosmetic mask in face, eye, finger, hand or foot shape.

## Revendications

1. Utilisation d'une fibre de viscose pour la fabrication d'un masque cosmétique transparent, **caractérisée en ce que**
- la fibre de viscose est une fibre plate ;
- la section transversale des fibres de viscose présente un rapport largeur/épaisseur qui est compris entre 6:1 et 30:1 ; et
- le titre des fibres de viscose est compris entre 1,0 dtex et 4 dtex.

2. Utilisation d'une fibre de viscose selon la revendication 1, **caractérisée en ce que** la section transversale présente un rapport largeur/épaisseur qui est compris entre 10:1 et 30:1, de préférence entre 13:1 et 20:1, de manière encore plus préférée entre 15:1 et 19:1 et de manière particulièrement préférée entre 17:1 et 18:1.

3. Utilisation d'une fibre de viscose selon la revendication 1 ou 2, **caractérisée en ce que** le titre de la fibre ou des fibres de viscose est compris entre 2,0 dtex et 4,2 dtex, de préférence entre 2,2 dtex et 3,8 dtex, de manière particulièrement préférée entre 2,4 dtex et 3,0 dtex, en particulier entre 2,4 dtex et 2,5 dtex.

4. Utilisation d'une fibre de viscose selon l'une quelconque des revendications 2 ou 3, **caractérisée en ce que** la fibre de viscose est sensiblement lisse.

5. Utilisation d'une fibre de viscose selon l'une quelconque des revendications 2 à 4, **caractérisée en ce que** la fibre de viscose est sensiblement transparente.

6. Utilisation d'une fibre de viscose selon la revendication 1, **caractérisée en ce que** la section transversale présente un rapport largeur/épaisseur qui est compris entre 6:1 et 15:1, de préférence entre 8:1 et 10:1 et de manière particulièrement préférée entre 8:1 et 9:1.

7. Utilisation d'une fibre de viscose selon la revendication 6, **caractérisée en ce que** le titre de la fibre ou des fibres de viscose est compris entre 1,0 dtex et 3,0 dtex, de préférence entre 1,7 dtex et 2,4 dtex, de manière particulièrement préférée entre 1,8 dtex et moins de 2,0 dtex, en particulier entre 1,8 dtex et 1,9 dtex.

8. Utilisation d'une fibre de viscose selon l'une quelconque des revendications précédentes, **caractérisée en ce qu'**elle est utilisée pour la fabrication d'un masque cosmétique sous la forme d'une feuille (non tissée) imprégnée de lotion.

9. Utilisation d'une fibre de viscose selon l'une quelconque des revendications précédentes, **caractérisée en ce qu'**elle est composée à plus de 98 % (p/p) de cellulose.

10. Utilisation d'une fibre de viscose selon l'une quelconque des revendications 1 à 9, **caractérisée en ce qu'**elle contient un polymère absorbant dans une proportion comprise entre 2 % et 20 % (p/p), de préférence entre 3 % et 10 % (p/p), de manière particulièrement préférée entre 4 % et 7 % (p/p).

11. Utilisation selon la revendication 10, **caractérisée en ce que** le polymère absorbant est un polymère qui est choisi dans le groupe constitué par le carboxyméthylcellulose (CMC), les amidons modifiés ainsi que leurs mélanges.

12. Utilisation d'une fibre de viscose selon l'une quelconque des revendications précédentes, **caractérisée en ce qu'**elle présente une longueur de fibre qui est comprise entre 2 mm et 80 mm.

13. Utilisation d'une fibre de viscose selon l'une quelconque des revendications précédentes, ladite fibre étant destinée à la fabrication d'un masque cosmétique en forme de visage, d'yeux, de doigt, de main ou de pied.
